# EUROPEAN PATENT SPECIFICATION

(11) **EP 2 489 652 B1**
(45) Date of publication and mention of the grant of the patent: **25.07.2018**
(21) Application number: 10823202.6
(22) Date of filing: 14.10.2010
(51) Int. Cl.: A61K 36/82, A61K 36/15, A61K 36/53, A61K 36/736, A61K 9/48, A61K 31/09, A61K 31/122, C07C 41/26, A61K 47/04, A61K 47/10, A61K 47/12, A61K 47/14, A61K 47/44, A61K 47/46, A61P 17/18, A61P 39/06, C07C 41/46, C07C 43/23

(54) **METHOD FOR PRODUCING REDUCED COENZYME Q10, METHOD FOR STABILIZING SAME, AND COMPOSITION COMPRISING SAME**
VERFAHREN ZUR HERSTELLUNG VON REDUZIERTEM COENZYM Q10, VERFAHREN ZU SEINER STABILISIERUNG UND ZUSAMMENSETZUNG DAMIT
PROCÉDÉ DE PRODUCTION DE COENZYME Q10 RÉDUITE, PROCÉDÉ POUR SA STABILISATION, ET COMPOSITION EN COMPORTANT

(30) Priority: 16.10.2009 JP 2009239741
(43) Date of publication of application: 22.08.2012
(73) Proprietor: Kaneka Corporation, Osaka (JP)
(72) Inventor: YAMAGUCHI, Takao, Takasago-shi Hyogo 676-8688 (JP); JIKIHARA, Takaaki, Takasago-shi Hyogo 676-8688 (JP); KITAMURA, Shiro, Takasago-shi Hyogo 676-8688 (JP); UEDA, Yasuyoshi, Takasago-shi Hyogo 676-8688 (JP)
(74) Representative: Vossius & Partner Patentanwälte Rechtsanwälte mbB
(86) International application number: PCT/JP2010/006103
(87) International publication number: WO 2011/045934

(56) References cited:
- EP-A1- 1 452 174
- EP-A1- 2 067 406
- WO-A1-03/062182
- WO-A1-2005/035477
- WO-A1-2009/025277
- WO-A1-2011/029942
- JP-A- 2003 119 126
- JP-A- 2007 055 980
- JP-A- 2008 273 963
- DATABASE WPI Week 200880 Thomson Scientific, London, GB; AN 2008-N83585 XP002722502, & WO 2008/129980 A1 (KANEKA CORP) 30 October 2008 (2008-10-30)
- KUBO ET AL: "Food content of ubiquinol-10 and ubiquinone-10 in the Japanese diet", JOURNAL OF FOOD COMPOSITION AND ANALYSIS, ACADEMIC PRESS, LONDON, GB, vol. 21, no. 3, 21 November 2007 (2007-11-21), pages 199-210, XP022517673, ISSN: 0889-1575, DOI: 10.1016/J.JFCA.2007.10.003
- BELTRAN E ET AL: "Use of antioxidants to minimize rancidity in pressurized and cooked chicken slurries", MEAT SCIENCE, ELSEVIER SCIENCE, GB, vol. 66, no. 3, 1 March 2003 (2003-03-01), pages 719-725, XP002670713, ISSN: 0309-1740, DOI: 10.1016/J.MEATSCI.2003.07.004 [retrieved on 2003-09-23]

## Description

### Technical Field

The present invention relates to a production method of reduced coenzyme Q10, a stabilizing method thereof and a composition containing the same. As compared to oxidized, coenzyme Q10, reduced coenzyme Q10 shows high oral absorbability, and is a compound useful , e.g., as a superior food, health food, food with nutrient function claims, food for specified health use, supplement, nutritional supplement, nutritional product, drink, feed, animal drug, cosmetic, pharmaceutical product, therapeutic drug, and prophylactic drug.

### Background Art

As compared to oxidized coenzyme Q10, reduced coenzyme Q10 shows high oral absorbability and is a compound highly useful as an antioxidant substance.

Reduced coenzyme Q10 can be obtained, for example, by subjecting oxidized coenzyme Q10 obtained by a conventionally known method such as synthesis, fermentation, extraction from a naturally-occurring substance to a reduction reaction (patent document 1).

As a component (reducing agent) effective for reducing oxidized coenzyme Q10 to reduced coenzyme Q10, sodium borohydride (non-patent document 1), sodium dithonite (non-patent document 2), sulfite type substance (sodium sulfite etc.) (non-patent document 3), ascorbic acids and many other components (patent document 2) have been reported.

However, these components do not necessarily satisfy preferably the requested conditions, since they are compounds requiring attention in production and handling because they have a risk of ignition, are expensive, are associated with a concern about safety to the body, and are influential on the flavor when used for food. In many cases, a step of removing reduced coenzyme Q10 from the above-mentioned reducing agent and by-products thereof by separation after completion of the reduction reaction is required. There is a strong demand, therefore, on a component whose reducing agent and by-product can be directly used as a component effective for stabilization, or applicable, e.g., to food, health food, and supplement, after completion of the reduction reaction, has been desired.

Among the components effective for reducing oxidized coenzyme Q10 are those effective for stabilizing reduced coenzyme Q10, i.e., those utilizable as antioxidants.

It is known that reduced coenzyme Q10 is easily oxidized by oxygen in the air to give oxidized coenzyme Q10. Thus, a method of stabilizing a preparation and a combination agent of reduced coenzyme Q10 by protecting them from oxidization is extremely important.

As a compound effective for stabilizing reduced coenzyme Q10, citric acid and ascorbic acids are known (patent document 3). In addition, as a solvent for stabilizing reduced coenzyme Q10, hydrocarbons, fatty acid esters, ethers and nitriles are known to be preferable (patent document 4).

However, these components and solvents effective for stabilizing reduced coenzyme Q10 do not necessarily satisfy preferably the requested conditions, since the components and solvents themselves or by-products thereof are, e.g., associated with a concern about safety to the body, influential on the flavor when used for food, and are expensive, and further stabilization is sometimes desired depending on the needs.

Under such background, a component that can be used as a component effective for reducing oxidized coenzyme Q10 and/or a component effective for stabilizing reduced coenzyme Q10, and preferably satisfies characteristics of low risk in production and handling, and being economical and functional has been strongly desired.

Patent document 5 relates to a composition comprising reduced coenzyme Q 10 and an anthocyanin extract.

Patent document 6 relates to a specific particulate composition which contains reduced coenzyme Q10 and a specific method for stabilizing the particulate composition.

Patent document 7 relates to a milk or dairy product which contains an oil/fat and is enriched with ubiquinol.

Patent document 8 relates to a method of stabilizing reduced coenzyme Q10 which comprises subjecting reduced coenzyme Q10 to be coexisted with citric acid or a related compound thereof.

Non-patent document 4 relates to determining the ubiquinol-10 and ubiquinone-10 content of various foods and using the data on the consumption of foods to estimate the average dietary intake of ubiquinol-10 and of total CoQ₁₀ in the Japanese population. Non-patent document 5 relates to the testing of the role of several antioxidants in chicken slurries submitted to several pressure conditions and/or cooked.

### [Document List]

### [Patent documents]

patent document 1: JP-A-H10-109933
patent document 2: WO01/52822
patent document 3: WO03/032967
patent document 4: WO03/006408
patent document 5: WO11/029942
patent document 6: WO08/129980
patent document 7: EP-A1-2 067 406
patent document 8: EP-A1-1 452 174

### [non-patent documents]

non-patent document 1: Journal of Applied Toxicology, 28(1), 55-62, 2008
non-patent document 2: Pharmaceutical Research, 23(1), 70-81, 2006
non-patent document 3: the 7th edition of Japanese Standards of Food Additives
non-patent document 4: Journal of food composition and analysis, 21(3), 199-210, 2007
non-patent document 5: Meat Science, 66(3), 719-725, 2003

### [Summary Of The Invention]

### Problems to be Solved by the Invention

In view of the above, the present invention aims to provide a production method of reduced coenzyme Q10 utilizing a component that is highly safe, easily applicable , for example, to food, health food, food with nutrient function claims, food for specified health use, supplement, nutritional supplement, nutritional product, animal drug, drink, feed, pet food, cosmetic, pharmaceutical product, therapeutic drug, and prophylactic drug, and does not need to be removed by separation after production, further a preferable method capable of stabilizing the reduced coenzyme Q10 by protecting from oxidation, and a stabilized composition.

### Means of Solving the Problems

The present inventors have conducted intensive studies and clarified that conventionally-reported reducing agents of oxidized coenzyme Q10 include many that in deed have poor reducing ability and are not practical. On the other hand, they have surprisingly found that a particular component derived from a naturally-occurring substance, which has not been conventionally considered to have a reducing ability, has an ability to reduce oxidized coenzyme Q10 to produce reduced coenzyme Q10, and reduced coenzyme Q10 is preferably protected from oxidization by a molecular oxygen in the presence of the particular component derived from a naturally-occurring substance, which resulted in the completion of the present invention.

Accordingly, the present invention relates to a production method of reduced coenzyme Q10, comprising reducing oxidized coenzyme Q10 by using a component derived from a naturally-occurring substance, wherein the component derived from a naturally-occurring substance is any one or more selected from the group consisting of an acerola extract, a tea extract, a rosemary extract, a pine bark extract and a Vaccinium vitis-idaea extract.

Furthermore, the present invention also relates to a method of stabilizing reduced coenzyme Q10, comprising co-presence of reduced coenzyme Q10 and an acerola extract and/or a Vaccinium vitis-idaea extract, as a component effective for stabilization of reduced coenzyme Q10. The present invention is also directed to a composition containing reduced coenzyme Q10 and acerola extract and/or a Vaccinium vitis-idaea extract as defined in claim 9.

### Effect of the Invention

According to the present invention, a convenient production method of reduced coenzyme Q10, which comprises reducing oxidized coenzyme Q10 by using a component safe for the body, can be provided, and a stabilizing method of reduced coenzyme Q10 which uses said component can also be provided. Furthermore, since such component effective for reducing oxidized coenzyme Q10 and/or a component effective for stabilizing reduced coenzyme Q10, which are/is used in the present invention, can also be expected to function as a nutrition and supplement material. Therefore, a composition obtained by the above-mentioned production method and/or the above-mentioned stabilization method is extremely useful particularly as a pharmaceutical product, supplement, food with nutrient function claims, food for specified health uses, nutritional supplement, nutritional product, animal drug, cosmetic, or therapeutic drug, which are required to have various effects.

### [Description of Embodiments]

The present invention is explained in detail in the following. First, the production method of reduced coenzyme Q10 of the present invention is explained. The production method of the present invention is a production method of reduced coenzyme Q10 comprising reducing oxidized coenzyme Q10 by using a particular component derived from a naturally-occurring substance.

Oxidized coenzyme Q10 to be a starting material in the production method of the present invention may be oxidized coenzyme Q10 alone or a mixture thereof with reduced coenzyme Q10. When the above-mentioned oxidized coenzyme Q10 is a mixture thereof with reduced coenzyme Q10, the ratio of oxidized coenzyme Q10 in the total amount of coenzyme Q10 (i.e., total amount of reduced coenzyme Q10 and oxidized coenzyme Q10) is not particularly limited and is, for example, not less than 1 wt%, normally not less than 5 wt%, preferably not less than 10 wt%, more preferably not less than 20 wt%, further preferably not less than 50 wt%, particularly preferably not less than 60 wt%, most preferably not less than 80 wt%. While the upper limit is not particularly limited, when a mixture with reduced coenzyme Q10 is used as oxidized coenzyme Q10, it is normally not more than 99.9 wt%. Needless to say, when oxidized coenzyme Q10 is 100 wt%, oxidized coenzyme Q10 alone can be used. Oxidized coenzyme Q10 to be used here can be obtained by a conventionally known method, for example, synthesis, fermentation, or extraction from a naturally-occurring substance. Preferably, it is obtained by fermentation or extraction from a naturally-occurring substance.

In the production method of the present invention, a particular component derived from a naturally-occurring substance is used as a component for reducing oxidized coenzyme Q10 to give reduced coenzyme Q10. The component derived from a naturally-occurring substance, which is used for reduction of oxidized coenzyme Q10 in the production method of the present invention, is an acerola extract, a tea extract, a rosemary extract, a pine bark extract or a Vaccinium vitis-idaea extract (hereinafter these 5 kinds are sometimes to be generically referred to as "the component derived from a naturally-occurring substance useful in the present invention"). Here, while the tea extract is not particularly limited, specific examples include green tea extract, oolong tea extract, and ten tea extract. As the pine bark extract, for example, commercially available pycnogenol, and flavangenol
can also be used. Vaccinium vitis-idaea to be the starting material of Vaccinium vitis-idaea extract may be any of Cowberry and Lingonberry.

While any of the above-mentioned components derived from naturally-occurring substances useful in the present invention is an extract from a plant, the extraction method thereof is not particularly limited. Extracts obtained by a known extraction method can be utilized, of which an extract with actual use performance as a supplement material is preferably used. Any of the above-mentioned components derived from a naturally-occurring substance may be used alone, or two or more kinds may be used in combination.

Among the above-mentioned components derived from a naturally-occurring substance, an acerola extract or a Vaccinium vitis-idaea extract is preferable for the production method of the present invention, in view of the reducing ability thereof or effectiveness of the component itself.

In the production method of the present invention, the weight ratio of the above-mentioned component derived from a naturally-occurring substance useful in the present invention to oxidized coenzyme Q10 (when plural components are used in combination as a component derived from a naturally-occurring substance, a weight ratio of the total amount thereof) is not particularly limited as long as it is an amount effective for reducing oxidized coenzyme Q10 to reduced coenzyme Q10. Generally, the weight ratio of the component derived from a naturally-occurring substance to oxidized coenzyme Q10, that is, weight of the component derived from a naturally-occurring substance to be used/weight of oxidized coenzyme Q10 to be the starting material, is normally about 1/1000 or more, preferably about 1/100 or more, more preferably about 1/10 or more, particularly preferably about 1/1 or more. While its upper limit is not particularly limited, it is about 10000/1 or less, preferably about 1000/1 or less, more preferably about 100/1 or less, particularly preferably about 10/1 or less, from the aspects of economic aspect and effectiveness as a nutrient.

In the production method of the present invention, oxidized coenzyme Q10 to be the starting materials and the above-mentioned component derived from a naturally-occurring substance useful in the present invention only need to be in contact with each other in the reaction system, where the system may be homogeneous or nonhomogeneous and is not particularly limited. For example, it may be a reaction system wherein oxidized coenzyme Q10 and the component derived from a naturally-occurring substance useful in the present invention are in contact with each other as a solid, wherein one is present in a liquid layer and dissolved in a solvent, and the other is present as a solid in the liquid layer, wherein oxidized coenzyme Q10 is present as a melt, and the component derived from a naturally-occurring substance useful in the present invention is present as a solid in the melt, wherein both are present in different phases, forming liquid-liquid two layers, wherein both are present in the same liquid phase. Needless to say, a system showing high contact efficiency between oxidized coenzyme Q10 and the component derived from a naturally-occurring substance useful in the present invention is effective for the reduction of oxidized coenzyme Q10. From this aspect, most preferred is the coexistence of oxidized coenzyme Q10 and the component derived from a naturally-occurring substance useful in the present invention in the same liquid phase.

From the above aspects, when a reduction reaction is performed in the production method of the present invention, a solvent is preferably used to allow presence of oxidized coenzyme Q10 and/or the component derived from a naturally-occurring substance useful in the present invention in the liquid phase. The solvent to be used for reduction reaction in the production method of the present invention is not particularly limited, and organic solvents such as hydrocarbons, fatty acid esters, ethers, alcohols, ketones, nitrogen compounds (including nitriles, amides), sulfur compounds, fatty acids, and terpenes, fats, oils, and water can be
mentioned. These may be used alone or as a mixed solvent of 2 or more kinds thereof.

While the above-mentioned hydrocarbons are not particularly limited, for example, aliphatic hydrocarbon, aromatic hydrocarbon, and halogenated hydrocarbon can be mentioned. Particularly, aliphatic hydrocarbon and aromatic hydrocarbon are preferable, and aliphatic hydrocarbon is especially preferable.

While aliphatic hydrocarbon may be cyclic or acyclic, saturated or unsaturated and is not particularly limited, acyclic aliphatic hydrocarbon is particularly preferably used. In addition, aliphatic hydrocarbon having 3 to 20 carbon atoms, preferably 5 to 12 carbon atoms, can be generally used.

Specific examples of the aliphatic hydrocarbon include propane, butane, isobutane, pentane, 2-methylbutane, cyclopentane, 2-pentene, hexane, 2-methylpentane, 2,2-dimethylbutane, 2,3-dimethylbutane, methylcyclopentane, cyclohexane, 1-hexene, cyclohexene, heptane, 2-methylhexane, 3-methylhexane, 2, 3-dimethylpentane, 2,4-dimethylpentane, methylcyclohexane, 1-heptene, octane, 2,2,3-trimethylpentane, isooctane, ethylcyclohexane, 1-octene, nonane, 2,2,5-trimethylhexane, 1-nonene, decane, 1-decene, p-menthane, undecane, and dodecane.

Among these, saturated aliphatic hydrocarbon having 5 to 8 carbon atoms is preferable, and pentane, 2-methylbutane, cyclopentane, hexane, 2-methylpentane, 2,2-dimethylbutane, 2,3-dimethylbutane, methylcyclopentane, cyclohexane, heptane, 2-methylhexane, 3-methylhexane, 2,3-dimethylpentane, 2,4-dimethylpentane, methylcyclohexane, octane, 2,2,3-trimethylpentane, isooctane, and ethylcyclohexane are particularly preferable.

While the aromatic hydrocarbon is not particularly limited, normally, aromatic hydrocarbon having 6 to 20 carbon atoms, particularly 6 to 12 carbon atoms, especially 7 to 10 carbon atoms, is preferably used. Specific examples of the aromatic hydrocarbon include benzene, toluene, xylene, o-xylene, m-xylene, p-xylene, ethylbenzene, cumene, mesitylene, tetralin, butylbenzene, p-cymene, cyclohexylbenzene, diethylbenzene, pentylbenzene, dipentylbenzene, dodecylbenzene, and styrene.

It is preferably toluene, xylene, o-xylene, m-xylene, p-xylene, ethylbenzene, cumene, mesitylene, tetralin, butylbenzene, p-cymene, cyclohexylbenzene, diethylbenzene or pentylbenzene, more preferably, toluene, xylene, o-xylene, m-xylene, p-xylene, cumene or tetralin, and most preferably cumene.

The halogenated hydrocarbon may be cyclic or acyclic, saturated or unsaturated, and is not particularly limited. In general, acyclic one is preferably used. Normally, chlorinated hydrocarbon and fluorinated hydrocarbon are preferable, and chlorinated hydrocarbon is particularly preferable. A halogenated hydrocarbon having 1 to 6 carbon atoms, particularly 1 to 4 carbon atoms, especially 1 or 2 carbon atoms, is preferably used.

Specific examples of the halogenated hydrocarbon include dichloromethane, chloroform, carbon tetrachloride, 1,1-dichloroethane, 1,2-dichloroethane, 1,1,1-trichloroethane, 1,1,2-trichloroethane, 1,1,1,2-tetrachloroethane, 1,1,2,2-tetrachloroethane, pentachloroethane, hexachloroethane, 1,1-dichloroethylene, 1,2-dichloroethylene, trichloroethylene, tetrachloroethylene, 1,2-dichloropropane, 1,2,3-trichloropropane, chlorobenzene, and 1,1,1,2-tetrafluoroethane.

It is preferably dichloromethane, chloroform, carbon tetrachloride, 1,1-dichloroethane, 1,2-dichloroethane, 1,1,1-trichloroethane, 1,1,2-trichloroethane, 1,1-dichloroethylene, 1,2-dichloroethylene, trichloroethylene, chlorobenzene or 1,1,1,2-tetrafluoroethane, more preferably dichloromethane, chloroform, 1,2-dichloroethylene, trichloroethylene, chlorobenzene or 1,1,1,2-tetrafluoroethane.

While the above-mentioned fatty acid esters are not particularly limited, for example, propionate, acetate, and formate
can be mentioned. Particularly, acetate and formate are preferable, and acetate is especially preferable. While ester group is not particularly limited, in general, alkyl ester or aralkyl ester having 1 to 8 carbon atoms, preferably alkyl ester having 1 to 6 carbon atoms, more preferably alkyl ester having 1 to 4 carbon atoms, is preferably used.

Examples of propionate include methyl propionate, ethyl propionate, butyl propionate and isopentyl propionate.

Examples of acetate include methyl acetate, ethyl acetate, propyl acetate, isopropyl acetate, butyl acetate, isobutyl acetate, sec-butyl acetate, pentyl acetate, isopentyl acetate, sec-hexyl acetate, cyclohexyl acetate, and benzyl acetate.

It is preferably methyl acetate, ethyl acetate, propyl acetate, isopropyl acetate, butyl acetate, isobutyl acetate, sec-butyl acetate, pentyl acetate, isopentyl acetate, sec-hexyl acetate or cyclohexyl acetate, more preferably methyl acetate, ethyl acetate, propyl acetate, isopropyl acetate, butyl acetate or isobutyl acetate, and most preferably ethyl acetate.

Examples of formate include methyl formate, ethyl formate, propyl formate, isopropyl formate, butyl formate, isobutyl formate, sec-butyl formate, and pentyl formate. It is preferably methyl formate, ethyl formate, propyl formate, butyl formate, isobutyl formate or pentyl formate, and most preferably ethyl formate.

The above-mentioned ethers may be cyclic or acyclic, saturated or unsaturated, and are not particularly limited. Generally, saturated ones are preferably used. Normally, ether having 3 to 20 carbon atoms, particularly 4 to 12 carbon atoms, especially 4 to 8 carbon atoms, is preferably used.

Specific examples of the ethers include diethyl ether, methyl tert-butyl ether, dipropyl ether, diisopropyl ether, dibutyl ether, dihexyl ether, ethylvinyl ether, butylvinyl ether, anisole, phenetole, butylphenyl ether, methoxytoluene, dioxane, furan, 2-methylfuran, tetrahydrofuran, tetrahydropyran, ethylene glycol dimethyl ether, ethylene glycol diethyl ether, ethylene glycol dibutyl ether, ethylene glycol monomethyl ether, ethylene glycol monoethyl ether, and ethylene glycol dibutyl ether.

Preferred as the ethers are diethyl ether, methyl tert-butyl ether, dipropyl ether, diisopropyl ether, dibutyl ether, dihexyl ether, anisole, phenetole, butylphenyl ether, methoxytoluene, dioxane, 2-methylfuran, tetrahydrofuran, tetrahydropyran, ethylene glycol dimethyl ether, ethylene glycol diethyl ether, ethylene glycol dibutyl ether, ethylene glycol monomethyl ether and ethylene glycol monoethyl ether, more preferred are diethyl ether, methyl tert-butyl ether, anisole, dioxane, tetrahydrofuran, ethylene glycol monomethyl ether, ethylene glycol monoethyl ether, more.preferably, diethyl ether, methyl tert-butyl ether, and anisole, and most preferably, methyl tert-butyl ether.

The above-mentioned alcohols may be cyclic or non-cyclic, saturated or unsaturated, and are not particularly limited, and generally, saturated ones are preferably used. Normally, a monovalent alcohol having 1 to 20 carbon atoms, particularly 1 to 12 carbon atoms, especially 1 to 6 carbon atoms, among others 1 to 5 carbon atoms, is preferable, a divalent alcohol having 2 to 5 carbon atoms is preferable, and a trivalent alcohol having 3 carbon atoms is preferable.

Examples of the monovalent alcohol include methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol, 1-heptanol, 2-heptanol, 3-heptanol, 1-octanol, 2-octanol, 2-ethyl-1-hexanol, 1-nonanol, 1-decanol, 1-undecanol, 1-dodecanol, allyl alcohol, propargyl alcohol, benzyl alcohol, cyclohexanol, 1-methylcyclohexanol, 2-methylcyclohexanol, 3-methylcyclohexanol, and 4-methylcyclohexanol.

Preferred as the monovalent alcohol are methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol and cyclohexanol, more preferred are methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, further preferably, methanol, ethanol, 1-propanol, 2-propanol, 1-butanol, 2-butanol, isobutyl alcohol, 2-methyl-1-butanol, isopentyl alcohol, and most preferred is ethanol.

Examples of the divalent alcohol include 1,2-ethanediol, 1,2-propanediol, 1,3-propanediol, 1,2-butanediol, 1,3-butanediol, 1, 4-butanediol, 2, 3-butanediol, and 1,5-pentanediol.

Preferred are 1,2-ethanediol, 1,2-propanediol and 1,3-propanediol, and most preferred is 1,2-ethanediol.

As the trivalent alcohol, glycerol can be preferably used.

The above-mentioned ketones are not particularly limited, and normally, ketone having 3 to 6 carbon atoms is preferably used. Specific examples of the ketones include acetone, methyl ethyl ketone, methyl butyl ketone, and methyl isobutyl ketone.

Preferred are acetone and methyl ethyl ketone, and most preferred is acetone.

Nitriles may be cyclic or acyclic, saturated or unsaturated, and is not particularly limited. In general, saturated one is preferably used. Normally, nitrile having 2 to 20 carbon atoms, particularly 2 to 12 carbon atoms, especially 2 to 8 carbon atoms, is preferably used. Specific examples of the nitriles include acetonitrile, propionitrile, malononitrile, butyronitrile, isobutyronitrile, succinonitrile, valeronitrile, glutaronitrile, hexanenitrile, heptyl cyanide, octyl cyanide, undecanenitrile, dodecanenitrile, tridecanenitrile, pentadecanenitrile, stearonitrile, chloroacetonitrile, bromoacetonitrile, chloropropionitrile, bromopropionitrile, methoxyacetonitrile, methyl cyanoacetate, ethyl cyanoacetate, tolunitrile, benzonitrile, chlorobenzonitrile, bromobenzonitrile, cyanobenzoic acid, nitrobenzonitrile, anisonitrile, phthalonitrile, bromotolunitrile, methylcyanobenzoate, methoxybenzonitrile, acetylbenzonitrile, naphtonitrile, biphenylcarbonitrile, phenylpropionitrile, phenylbutyronitrile, methylphenylacetonitrile, diphenylacetonitrile, naphthylacetonitrile, nitrophenylacetonitrile, chlorobenzyl cyanide, cyclopropanecarbonitrile, cyclohexanecarbonitrile, cycloheptanecarbonitrile, phenylcyclohexanecarbonitrile, and tolylcyclohexanecarbonitrile. It is preferably acetonitrile, propionitrile, succinonitrile, butyronitrile, isobutyronitrile, valeronitrile, methyl cyanoacetate, ethyl cyanoacetate, benzonitrile, tolunitrile or chloropropionitrile, more preferably acetonitrile, propionitrile, butyronitrile or isobutyronitrile, most preferably acetonitrile.

Examples of the nitrogen compounds other than nitriles include nitromethane, acetonitrile, triethylamine, pyridine, formamide, N-methylformamide, N,N-dimethylformamide, N,N-dimethylacetamide, N-methylpyrrolidone and the like.

Examples of the sulfur compounds include dimethyl sulfoxide, and sulfolane.

Examples of the above-mentioned fatty acids include formic acid, acetic acid, propionic acid, caprylic acid, capric acid, lauric acid, myristic acid, palmitic acid, stearic acid, isostearic acid, oleic acid, linoleic acid, linolenic acid, arachidonic acid, behenic acid, eicosapentaenoic acid, docosahexaenoic acid, and docosapentaenoic acid, formic acid, acetic acid,' caprylic acid, capric acid, oleic acid, linoleic acid, linolenic acid, arachidonic acid, eicosapentaenoic acid, docosahexaenoic acid, or docosapentaenoic acid is preferable, particularly, acetic acid, oleic acid, linoleic acid, linolenic acid, arachidonic acid, eicosapentaenoic acid, docosahexaenoic acid, or docosapentaenoic acid is preferable, furthermore, oleic acid, linoleic acid, linolenic acid, eicosapentaenoic acid, or docosahexaenoic acid is preferable, and most preferably is oleic acid.

The above-mentioned terpenes may be cyclic or acyclic, or saturated or unsaturated, and are not particularly limited. Generally, hemiterpene, monoterpene, sesquiterpene, diterpene, triterpene, and tetraterpene can be mentioned.

Specific examples of the terpenes include purenoru, 3-methyl-3-buten-2-ol, tiglic acid, angelic acid, seneshio acid, isovaleric acid, alloocimene, α-bisabolene, bisabolene, β-bourbonene, δ-cadinene, δ-3-carene, α-caryophyllene, β-caryophyllene, p-cymene, dehydro-p-cymene, menthol, limonene, d-limonene, 1-limonene, cis-3,7-dimethyl-1,3,6,-octatriene, δ-elemene, β-elemene, α-farnesene, β-farnesene, farnesene, germacrene D, β-guaiene, longifolene, myrcene, β-ocimene, α-phellandrene, α-pinene, β-pinene, pinocamphone, sabinene, α-terpinene, γ-terpinene, terpinolen, thujopsis, valencene, α-copaene, hydrogenated limonene dimer, isocaryophyllene, pinene dimer, dipentene dimer, dipentene trimer, geraniol, citral, citronellal, citronellol, 1,8-cineol, hydroxycitronellal, linalool, cosmene, nerol, myrcenol, lavandulol, ipsdienol, neral, geranial, perylene, rose furan, geranyl acid, thioterpineol, α-terpineol, β-terpineol, γ-terpineol, δ-terpineol, carveol, thelepin, perillaldehyde, perillylalcohol, carvone, ascaridole, anethole, thujone, thujanol, α-ionone, β-ionone, γ-ionone, farnesol, nerolidol, α-sinensal, β-sinensal, bissabol, phytol, squalene, citronellioxyacetoaldehyde, myrtenal, perillaaldehyde, 2-p-cymenol, 2-ethoxy-p-cymene, carvenol, 4-carvomenthenol, carvyl acetate, carvyl propionate, caryophyllenealcohol, caryophyllenealcohol acetate, 1,4-cineol, eugenol, d-selinene, thymol, d-camphene, linaloolacetate.

Preferred as terpenes are purenoru, 3-methyl-3-butene-2-ol, tiglic acid, angelic acid, seneshio acid, isovaleric acid, alloocimene, α-bisabolene, bisabolene, β-bourbonene, δ-cadinene, δ-3-carene, α-caryophyllene, β-caryophyllene, p-cymene, dehydro-p-cymene, limonene, d-limonene, 1-limonene, cis-3,7-dimethyl-1,3,6,-octatriene, δ-elemene, β-elemene, α-farnesene, β-farnesene, farnesene, germacrene D, β-guaiene, longifolene, myrcene, β-ocimene, α-phellandrene, α-pinene, β-pinene, pinocamphone, sabinene, α-terpinene, γ-terpinene, terpinolen, thujopsis, valencene, α-copaene, hydrogenated limonene dimer, isocaryophyllene, pinene dimer, dipentene dimer, dipentene trimer, geraniol, citral, citronellal, citronellol, 1,8-cineol, hydroxycitronellal, linalool, nerol, myrcenol, neral, geranial, carvone, anethole, thujone, phytol, squalene, eugenol, d-selinene, thymol and linalool acetate, particularly preferred are α-bisabolene, bisabolene, δ-cadinene, α-caryophyllene, β-caryophyllene, limonene, d-limonene, 1-limonene, myrcene, α-phellandrene, α-pinene, β-pinene, α-terpinene, γ-terpinene, geraniol, citral, citronellol, 1,8-cineol, linalool, carvone, anethole, thujone, eugenol, d-selinene, thymol and linalool acetate, and most preferred are limonene and d-limonene.

In addition, an essential oil containing the above-mentioned terpenes can be used as a solvent. While the essential oil is not particularly limited, orange oil, capsicum oil, mustard oil, garlic oil, callaway oil, clove oil, cinnamon oil, cocoa extract, coffee bean extract, ginger oil, spearmint oil, celery-seed oil, thyme oil, onion oil, nutmeg oil, parsley seed oil, mint oil, vanilla extract, fennel oil, pennyroyal oil, peppermint oil, eucalyptus oil, lemon oil, rose oil, rosemary oil, almond oil, ajowan oil, anise oil, amyris oil, angelica route oil, ambrette seed oil, estragon oil, origanum oil, orris root oil, olibanum oil, cassia oil, cascarilla oil, cananga oil, chamomile oil, calamus oil, cardamom oil, carrot seed oil, cubeb oil, cumin oil, grapefruit oil, cinnamon leaf oil, cade oil, pepper oil, costus root oil, congnac oil, copaiba oil, cilantro oil, perilla oil, musk, juniper berry oil, star anis oil, sage oil, savory oil, geranium oil, tangerin oil, dill oil, neroli oil, tolu balsam oil, basil oil, birch oil, patchouli oil, palmarosa oil, pimento oil, petitgrain oil, bay leaf oil, bergamot oil, peru balsam oil, benzoin resin, Bois de Rose oil, hop oil, boronia absolute, marjoram oil, mandarin oil, myrtle oil, Chinese lemon flavor, lime oil, lavandin oil, lavender oil, rue oil, lemongrass oil, lenthionine, lavage oil, laurel leaf oil, and worm wood oil can be mentioned.

Of the above-mentioned organic solvents, alcohols, fatty acids and terpenes are preferable, alcohols are more preferable, and among alcohols, ethanol is most preferable.

The above-mentioned fat or oil may be natural fat or oil from animals and vegetable, synthetic fat or oil, or processed fat or oil. Examples of the vegetable fat or vegetable oil include coconut oil, palm oil, palm kernel oil, flaxseed oil, camellia oil, brown rice germ oil, rape seed oil, rice oil, peanuts oil, corn oil, wheat germ oil, soybean oil, perilla oil, cottonseed oil, sunflower seed oil, kapok oil, evening primrose oil, shea butter, sal butter, cacao butter, sesame oil, safflower oil, olive oil, avocado oil, poppyseed oil,and burdock fruit oil. Examples of the animal fat and animal oil include lard, milk fat, fish oil, beef fat.

Furthermore, fat or oil (e.g., hydrogenated oil) obtained by processing the above by , for example, fractionation, hydrogenation, and transesterification can also be mentioned. Needless to say, for example, medium-chain triglyceride (MCT), and partial glycerides of fatty acid can also be used. In addition, a mixture thereof may be used.

The medium-chain triglyceride is not particularly limited and, for example, triglyceride wherein fatty acid has 6 to 12 carbon atoms, preferably 8 to 12 carbon atoms, can be mentioned.

Of the above-mentioned fats or oils, vegetable fat, vegetable oil, synthetic fat, synthetic oil, processed fat, and processed oil are preferable from the aspects of easy handling, and odor. Among these, specifically, coconut oil, palm oil, palm kernel oil, rape seed oil, rice oil, soybean oil, cottonseed oil, safflower oil, olive oil, medium-chain triglyceride (MCT), partial triglyceride of fatty acid and the like are preferable, and rice oil, soybean oil, rape seed oil, safflower oil, medium-chain triglyceride, and partial triglycerides of fatty acid are particularly preferable.

Among the above-mentioned solvents, those acceptable to foods, pharmaceutical products, and cosmetics are preferable, and those acceptable for foods are more preferable. In view of direct ingestion without processing of the reaction product and reactivity, alcohols, water, fats, oils, fatty acids, terpenes and mixtures thereof are preferable, and ethanol, fats and oils, terpenes and mixtures thereof are most preferable.

In the production method of the present invention, the reduction reaction of oxidized coenzyme Q10 to be the starting material and a component derived from a naturally-occurring substance useful in the present invention only needs to be performed in the presence of the above-mentioned solvent as necessary, and the method therefor is not limited.

In the production method of the present invention, a surfactant can also be added to the reaction system during the reduction reaction, and the addition is preferable in many cases.

Examples of the above-mentioned surfactant include, but are not limited to, glycerol fatty acid ester, sucrose ester of fatty acid, organic acid monoglyceride, sorbitan ester of fatty acid, polyoxyethylene sorbitan fatty acid ester, propylene glycol ester of fatty acid, condensed ricinoleic acid glycerides, saponin, and phospholipid.

Examples of the above-mentioned glycerol fatty acid ester include, but are not limited to, glycerol having a polymerization degree of 1 - 10. While the fatty acid residue constituting glycerol fatty acid ester is not particularly limited, fatty acid having a carbon number of 6 - 18 can be preferably used and, for example, caprylic acid, capric acid, lauric acid, myristic acid, palmitic acid, stearic acid, isostearic acid, oleic acid, linoleic acid, and linolenic acid
can be mentioned.

Examples of the above-mentioned sucrose ester of fatty acid include, but are not limited to, one wherein fatty acid having a carbon number of 6 - 22 is ester-bonded to one or more hydroxyl groups of sucrose, such as sucrose laurate, sucrose myristate, sucrose palmitate, sucrose stearate, sucrose oleate, sucrose behenate, and sucrose erucate.

Examples of the above-mentioned organic acid monoglyceride include, but are not limited to, caprylic acid and succinic acid ester of monoglycerol, stearic acid and citric acid ester of monoglycerol, stearic acid and acetic acid ester of monoglycerol, stearic acid and succinic acid ester of monoglycerol, stearic acid and lactic acid ester of monoglycerol, stearic acid and diacetyltartaric acid ester of monoglycerol, and oleic acid and citric acid ester of monoglycerol.

Examples of the above-mentioned sorbitan ester of fatty acid include, but are not limited to, sorbitan wherein one or more of the hydroxyl groups are ester bonded to fatty acid each having a carbon number of 6 - 18, such as sorbitan monolaurate, sorbitan monopalmitate, sorbitan monostearate, and sorbitan monooleate.

Examples of the above-mentioned polyoxyethylene sorbitan fatty acid ester include, but are not limited to, sorbitan polyoxyethylene monopalmitate, sorbitan polyoxyethylene monostearate, sorbitan polyoxyethylene monooleate, sorbitan polyoxyethylene tristearate and sorbitan polyoxyethylene trioleate, wherein 6 - 20 mol of an ethyleneoxide chain is added.

Examples of the above-mentioned polyglycerol ester of interesterified ricinoleic acid include, but are not limited to, one wherein polyglycerol has an average polymerization degree of 2 - 10, and polyricinoleic acid has an average condensation degree (average number of condensed ricinoleic acid) of 2 - 4, such as tetraglycerol condensed ricinoleate, pentaglycerol condensed ricinoleate, and hexaglycerol condensed ricinoleate.

As the above-mentioned propylene glycol ester of fatty acid, any of monoester or diester can be used. While the fatty acid residue constituting the propylene glycol ester of fatty acid is not particularly limited, fatty acid having a carbon number of 6 - 18 can be preferably used, and for example, caprylic acid, capric acid, lauric acid, myristic acid, palmitic acid, stearic acid, isostearic acid, oleic acid, linoleic acid, and linolenic acid can be mentioned.

Examples of the above-mentioned phospholipid include, but are not limited to, egg-yolk lecithin, purified soybean lecithin, phosphatidylcholine, phosphatidylethanolamine, phosphatidylserine, sphingomyelin, dicetyl phosphoric acid, stearylamine, phosphatidylglycerol, phosphatidic acid, phosphatidylinositolamine, cardiolipin, ceramide phosphoryl ethanolamine, ceramide phosphoryl glycerol and a mixture thereof. Needless to say, for example, a phospholipid after processing of hydrogenation, and enzyme degradation
can also be used. To improve absorbability of reduced coenzyme Q10, an enzymatically degraded phospholipid is preferably used.

Examples of the above-mentioned phospholipid include, but are not limited to, enju saponin, quillaja saponin, purification soybean saponin, and yucca saponin.

In the production method of the present invention, the concentration of oxidized coenzyme Q10 to the reaction system at the time of start of the reduction reaction (the total weight of the whole reaction mixture) is not particularly limited. It is normally about 0.01 wt% or more, preferably about 0.1 wt% or more, more preferably about 0.2 wt% or more, particularly preferably about 1 wt% or more, further preferably about 2 wt% or more and, among these, about 3 wt% or more.

While the reaction temperature of the reduction reaction in the production method of the present invention is not particularly limited, the reaction is performed generally at 20°C or more, preferably 30°C or more, more preferably 40°C or more, further preferably 50°C or more, particularly preferably 60°C or more, most preferably 75°C or more.

To exhibit the effect of the present invention at the maximum, the above-mentioned reduction reaction is preferably performed, for example, under a deoxygenation atmosphere. The deoxygenation atmosphere can be formed by inert gas replacement, reducing pressure, boiling and combining them. At least, inert gas replacement, that is, use of inert gas atmosphere, is preferable. Examples of the above-mentioned inert gas include nitrogen gas, helium gas, argon gas, hydrogen gas, and carbon dioxide gas, preferably nitrogen gas.

In the production method of the first of the present invention, the reduction reaction may be performed in a preparation. In other words, production of reduced coenzyme Q10 by preparing a mixture containing oxidized coenzyme Q10, the component derived from a naturally-occurring substance useful in the present invention and a solvent where necessary, processing the mixture into a preparation form, and reducing oxidized coenzyme Q10 to reduced coenzyme Q10 in the preparation is within the scope of the present invention. The reduction in this case is , for instance, performed by preservation over a predetermined period and heating. In the present invention, the preparation means an oral administration form such as capsule (hard capsule, soft capsule, microcapsule), tablet, syrup, and drink, or a form such as cream, suppository, and toothpaste. The preparation in which the reduction reaction is performed is preferably the above-mentioned oral administration form, which is more preferably capsule and particularly preferably soft capsule.

Reduced coenzyme Q10 can be conveniently produced by the production method of the present invention as mentioned above. Here, the ratio of reduced coenzyme Q10 to the total amount of coenzyme Q10 at the time of completion of the reaction (i.e., total amount of reduced coenzyme Q10 and oxidized coenzyme Q10) is normally about 5 wt% or more, preferably about 10 wt% or more, more preferably 20 wt% or more, particularly preferably 30 wt% or more, especially 40 wt% or more, most of all 50 wt% or more, and further, 70 wt% or more.

The reduced coenzyme Q10 obtained by the production method of the present invention can be obtained as crudely purified or pure reduced coenzyme Q10 by appropriately performing, after completion of the reduction reaction, removal of solvent and isolation and purification operation. The mixed composition after the completion of the reduction reaction can be directly used as a composition containing reduced coenzyme Q10 and the component derived from a naturally-occurring substance, or followed by formulation into a preparation, and used, for example, in the fields of pharmaceutical products, and foods.

Now the stabilization method of the present invention is explained. In the present invention, reduced coenzyme Q10 can be stabilized by the co-presence of reduced coenzyme Q10 and a particular component derived from a naturally-occurring substance in the composition.

In the stabilization method of the present invention, reduced coenzyme Q10 to be the object of stabilization may be reduced coenzyme Q10 alone, or a mixture thereof with oxidized coenzyme Q10. When the above-mentioned reduced coenzyme Q10 is a mixture thereof with oxidized coenzyme Q10, the ratio of reduced coenzyme Q10 to the total amount of coenzyme Q10 (i.e., total amount of reduced coenzyme Q10 and oxidized coenzyme Q10) is not particularly limited and, for example, it is 3 wt% or more, normally about 10 wt% or more, preferably about 20 wt% or more, more preferably 30 wt% or more, particularly 40 wt% or more, especially 50 wt% or more, most of all 60 wt% or more, and further, 80 wt% or more. While the upper limit is not particularly limited, it is normally 99.9 wt% or less. Needless to say, when reduced coenzyme Q10 is 100 wt%, reduced coenzyme Q10 alone can be used.

The reduced coenzyme Q10 to be used in the stabilization method of the present invention can be obtained, for example, by synthesis, fermentation, and extraction from a naturally-occurring substance,
or by utilizing a conventionally-known method such as reduction of oxidized coenzyme Q10.
Preferred are those obtained by reducing oxidized coenzyme Q10 such as existing high pure coenzyme Q10 , or a mixture of oxidized coenzyme Q10 and reduced coenzyme Q10 with a general reducing agent, such as sodium hydrosulfite (sodium dithionite), sodium borohydride, and ascorbic acids.
More preferred are those obtained by reducing oxidized coenzyme Q10 such as existing high pure coenzyme Q10 and the like, or a mixture of oxidized coenzyme Q10 and reduced coenzyme Q10 with ascorbic acids. In addition, needless to say, reduced coenzyme Q10 obtained by the above-mentioned production method of the present invention can also be used preferably.

The component derived from a naturally-occurring substance to be used in the stabilization method of the present invention is an acerola extract or a Vaccinium vitis-idaea extract. These two components may be used in combination. A detailed explanation of the Vaccinium vitis-idaea extract is the same as that presented for the above-mentioned production method of the present invention.

In the stabilization method of the present invention, the weight ratio of reduced coenzyme Q10 to be the target of stabilizing, and the above-mentioned acerola extract and/or Vaccinium vitis-idaea extract is not particularly limited. Generally, the weight ratio of the above-mentioned extract to reduced coenzyme Q10, that is, the ratio of total weight of the above-mentioned extract to be used/weight of reduced coenzyme Q10 is normally about 1/1000 or more, preferably about 1/100 or more, more preferably about 1/10 or more, particularly preferably about 1/1 or more. While the upper limit is not particularly set, it is about 10000/1 or less, preferably about 1000/1 or less, more preferably about 100/1 or less, particularly preferably about 10/1 or less.

In the stabilization method of the present invention, reduced coenzyme Q10 and an acerola extract and/or a Vaccinium vitis-idaea extract are co-present in the composition. Here, "co-present" only requests that the both be in contact in some form. The manner of contact is not particularly limited, and the system of the composition may be homogeneous or nonhomogeneous. For example, it may be reaction system wherein reduced coenzyme Q10 and an acerola extract and/or a Vaccinium vitis-idaea extract are in contact with each other as a solid, wherein one is present in a liquid layer and dissolved in a solvent, and the other is present as a solid in the liquid layer, wherein reduced coenzyme Q10 is present as a melt, and an acerola extract and/or a Vaccinium vitis-idaea extract is present as a solid in the melt, wherein both are present in different phases, forming liquid-liquid two layers, wherein both are present in the same liquid phase. Needless to say, a system showing high contact efficiency between reduced coenzyme Q10 and an acerola extract and/or a Vaccinium vitis-idaea extract is effective for stabilizing reduced coenzyme Q10. From this aspect, most preferred is the co-presence of reduced coenzyme Q10 and an acerola extract and/or a Vaccinium vitis-idaea extract in the same liquid phase.

From the above aspects, in the stabilization method of the present invention, a solvent is preferably co-present to allow presence, in the liquid phase, of reduced coenzyme Q10 and/or an acerola extract or a Vaccinium vitis-idaea extract in the composition. While the solvent to be used in the stabilization method of the present invention is not particularly limited, and specific and preferable examples thereof can be quoted from those mentioned for the production method of the present invention.

In addition, as in the production method of the present invention, a surfactant can also be further co-present or often preferably co-present in the stabilization method of the present invention. Examples of the detailed kind and preferable surfactants to be used in the stabilization method of the present invention are the same as those explained for the production method of the present invention.

In the stabilization method of the present invention, the method for allowing co-presence of reduced coenzyme Q10 and an acerola extract and/or a Vaccinium vitis-idaea extract is not particularly limited. For example, when reduced coenzyme Q10 added from the outside is used, the reduced coenzyme Q10 and an acerola extract and/or a Vaccinium vitis-idaea extract may be simply mixed or, after mixing them, a solvent as mentioned above may be further mixed therewith. Alternatively, a solution containing reduced coenzyme Q10 in the aforementioned solvent may be mixed with an acerola extract and/or a Vaccinium vitis-idaea extract, or a solution containing these extracts in the aforementioned solvent may be mixed with reduced coenzyme Q10, or a solution containing reduced coenzyme Q10 and a solution containing the extracts may be mixed.

Alternatively, reduced coenzyme Q10 obtained by the aforementioned production method of the present invention can be directly utilized, that is, a mixture of reduced coenzyme Q10 after completion of the reduction reaction and the component derived from a naturally-occurring substance of the present invention can be directly utilized for the stabilization method of the present invention, and this embodiment is one of the most preferable embodiments.

In the stabilization method of the present invention, as a substance other than reduced coenzyme Q10, an acerola extract and/or a Vaccinium vitis-idaea extract, and solvent and surfactant where necessary, for example, excipient, disintegrant, lubricant, binder, dye, anticoagulant, absorption promoter, solubilizing agent, stabilizer, flavor, and active ingredient other than reduced coenzyme Q10 can also be further co-present, without any particular limitation.

While the above-mentioned excipient is not particularly limited, for example, sucrose, lactose, glucose, starch, mannitol, crystalline cellulose, calcium phosphate, and calcium sulfate can be mentioned.

While the above-mentioned disintegrant is not particularly limited, for example, starch, agar, calcium citrate, calcium carbonate, carboxymethylcellulose, tragacanth, and alginic acid can be mentioned.

While the above-mentioned lubricant is not particularly limited, for example, talc, magnesium stearate, polyethylene glycol, silica, and hydrogenated oil can be mentioned.

While the above-mentioned binder is not particularly limited, for example, ethylcellulose, methylcellulose, hydroxypropylmethylcellulose, hydroxypropylcellulose, tragacanth, shellac, gelatin, pullulan, gum arabic, polyvinylpyrrolidone, polyvinyl alcohol, polyacrylic acid, polymethacrylic acid, and sorbitol can be mentioned.

While the above-mentioned dye is not particularly limited, for example, dye such as titanium oxide, synthesis colors, colcothar dye, and tar pigment can be mentioned.

While the above-mentioned anticoagulant is not particularly limited, for example, stearic acid, talc, light anhydrous silicic acid, and hydrated silicon dioxide
can be mentioned.

While the above-mentioned absorption promoter is not particularly limited, for example, higher alcohols, and higher fatty acids can be mentioned.

While the above-mentioned solubilizing agent for active ingredients is not particularly limited, for example, organic acids such as fumaric acid, succinic acid, and malic acid etc.,
can be mentioned.

While the above-mentioned stabilizer is not particularly limited, for example, benzoic acid, beeswax, hydroxypropylmethylcellulose, and methylcellulose can be mentioned.

While the above-mentioned flavor is not particularly limited, for example, orange oil, capsicum oil, mustard oil, garlic oil, caraway oil, clove oil, cinnamon oil, cocoa extract, coffee bean extract, ginger oil, spearmint oil, celery seed oil, thyme oil, onion oil, nutmeg oil, parsley seed oil, peppermint oil, vanilla extract, fennel oil, pennyroyal oil, peppermint oil, eucalyptus oil, lemon oil, rose oil, rosemary oil, almond oil, ajowan oil, anis oil, amyris oil, angelica route oil, ambrette seed oil, estragon oil, origanum oil, orris root oil, olibanum oil, quassia oil, cascarilla oil, cananga oil, chamomile oil, calamus oil, cardamom oil, carrot seed oil, cubeb oil, cumin oil, grapefruit oil, cinnamon leaf oil, cade oil, pepper oil, costus root oil, cognac oil, copaiba oil, cilantro oil, perilla oil, musk, juniper berry oil, star anise oil, sage oil, savory oil, geranium oil, tangerine oil, dill oil, neroli oil, tolu balsam oil, basil oil, birch oil, patchouli oil, palmarosa oil, pimento oil, petitgrain oil, bay leaf oil, bergamot oil, Peru balsam oil, benzoin resin, bois de rose oil, hops oil, Boronia Absolute, marjoram oil, mandarin oil, myrtle oil, Chinese lemon flavor, lime oil, lavandin oil, lavender oil, rue oil, lemongrass oil, lethionine, lovage oil, laurel leaf oil, and worm wood oil can be mentioned.

The above-mentioned substances may have plural roles. For example, starch may have roles of excipient and disintegrant.

In the stabilization method of the present invention, the content of an acerola extract and/or a Vaccinium vitis-idaea extract (total amount when the both are used in combination) relative to the total weight of the composition containing reduced coenzyme Q10, and an acerola extract and/or a Vaccinium vitis-idaea extract in co-presence is not particularly limited. To allow sufficient exertion of the stabilizing effect of reduced coenzyme Q10, however, the ratio relative to the total weight of the composition is normally about 0.01 wt% or more, preferably about 0.1 wt% or more, more preferably about 1 wt% or more, particularly preferably about 5 wt% or more. While the upper limit is not particularly set, it is normally about 99 wt% or less, preferably about 95 wt% or less, more preferably about 90 wt% or less, particularly preferably 80 wt% or less, from the economic aspect, for example, effectiveness as a nutrient.

In the stabilization method of the present invention, the content of reduced coenzyme Q10 relative to the total weight of the above-mentioned composition is not particularly limited. To ensure effectiveness of reduced coenzyme Q10 in the composition, it is normally about 0.001 wt% or more, preferably about 0.01 wt% or more, more preferably about 0.1 wt% or more, particularly preferably about 0.5 wt% or more. While the upper limit is not particularly set, it is preferably about 50 wt% or less, more preferably about 40 wt% or less, particularly preferably about 30 wt% or less.

To exert the maximum effects of the present invention, the stabilization method of the present invention is preferably performed by placing reduced coenzyme Q10 and an acerola extract and/or a Vaccinium vitis-idaea extract in co-presence under deoxygenation atmosphere. The co-presence under the deoxygenation atmosphere may be performed at any stage of preparation, preservation, processing into a preparation, and preservation after processing of the composition in the stabilization method of the present invention, or may be performed at plural or all stages. The deoxygenation atmosphere can be formed by inert gas replacement, reducing pressure, boiling and combining them. At least, inert gas replacement, that is, use of inert gas atmosphere, is preferable. Examples of the above-mentioned inert gas include nitrogen gas, helium gas, argon gas, hydrogen gas, and carbon dioxide gas, preferably nitrogen gas.

Now the composition of the present invention is explained. The composition of the present invention is a composition containing reduced coenzyme Q10, and an acerola extract and/or a Vaccinium vitis-idaea extract as defined in claim 9.

In the composition of the present invention, reduced coenzyme Q10, which is the active ingredient in the composition, is not only protected from oxidation by an acerola extract and/or a Vaccinium vitis-idaea extract and maintained stably, but also a component derived from a naturally-occurring substance superior in safety and effective as a nutrient is contained. Therefore, the composition is safe, expected to show a synergistic effect with reduced coenzyme Q10 and can also be a composition useful as, for example, foods and supplement such as food with nutrient function claims, and food for specified health uses, drinks, pharmaceutical products, animal drugs, cosmetics, and pet foods.

Moreover, when the composition of the present invention is produced by the above-mentioned production method of the present invention, high benefits are provided from the aspect of production, since not only oxidized coenzyme Q10, which is economical, can be utilized as a starting material, but also the component derived from a naturally-occurring substance useful in the present invention utilized for reduction of oxidized coenzyme Q10 does not require separation and removal after completion of the reduction reaction, since its safety to the body has been established, and the component derived from a naturally-occurring substance remaining in the composition can also be directly utilized for stabilization of reduced coenzyme Q10. Utilizing the present invention capable of obtaining a composition containing reduced coenzyme Q10 in situ, the production cost of a composition containing reduced coenzyme Q10 can be suppressed, and a composition containing reduced coenzyme Q10 can be provided at a low cost.

Specific examples and detailed explanation of reduced coenzyme Q10 to be contained in the composition of the present invention are the same as those explained for the stabilization method of the present invention. From the above-mentioned aspects, one obtained by reducing oxidized coenzyme Q10 with an acerola extract and/or a Vaccinium vitis-idaea extract is particularly preferable from the aspects of production.

In addition, the detailed explanation of the acerola extract and/or Vaccinium vitis-idaea extract to be contained in the composition of the present invention is the same as that for the production method and stabilization method of the present invention.

In the composition of the present invention, the amount ratio of reduced coenzyme Q10, and an acerola extract and/or a Vaccinium vitis-idaea extract that are contained in the composition is not particularly limited. Generally, as the ratio of the weight of reduced coenzyme Q10/total weight of the above-mentioned extracts, it is normally about 1000/1 or more, preferably about 100/1 or more, more preferably about 10/1 or more, particularly preferably about 1/1 or more, and about 1/10000 or less, preferably about 1/1000 or less, more preferably about 1/100 or less, particularly preferably about 1/10 or less.

In the composition of the present invention, reduced coenzyme Q10, and an acerola extract and/or a Vaccinium vitis-idaea extract are co-present in the composition. The "co-present" here means that they need to be in contact with each other in some form. The manner of contact is not particularly limited, and the system of the composition may be homogeneous or nonhomogeneous. Examples thereof include contact of reduced coenzyme Q10, and an acerola extract and/or a Vaccinium vitis-idaea extract each as a solid, the presence of one as a solid in a liquid layer of the other dissolved in a solvent and the like, the presence of an extract as a solid in a melt of reduced coenzyme Q10, each present in a different liquid phase, forming liquid-liquid two layers, and each present in the same liquid phase and the like. Needless to say, a system showing high contact efficiency of reduced coenzyme Q10, and an acerola extract and/or a Vaccinium vitis-idaea extract is effective for stabilization of reduced coenzyme Q10 and, from this aspect, reduced coenzyme Q10 and these extracts are most preferably present in the same liquid phase.

From the above-mentioned aspects, it is preferable in the composition of the present invention to use a solvent to contain reduced coenzyme Q10, and an acerola extract and/or a Vaccinium vitis-idaea extract in liquid phase in the composition. The solvent to be used for the composition of the present invention is not particularly limited, and specific examples, detailed kind and preferable examples thereof are the same as those mentioned for the aforementioned production method and stabilization method of the present invention.

In addition, as in the production method and stabilization method of the present invention, the composition of the present invention can also further contain or often preferably contain a surfactant. Specific examples, detailed kind and preferable examples of the surfactant to be used in the composition of the present invention are the same as those explained for the aforementioned production method and stabilization method of the present invention.

In the composition of the present invention, a method for preparing a composition containing reduced coenzyme Q10, and an acerola extract and/or a Vaccinium vitis-idaea extract is not particularly limited. For example, when reduced coenzyme Q10 added from outside is used, the reduced coenzyme Q10, and an acerola extract and/or a Vaccinium vitis-idaea extract may be simply mixed or, after mixing them, a solvent as mentioned above may be further mixed therewith. Alternatively, a solution containing reduced coenzyme Q10 in the aforementioned solvent may be mixed with an acerola extract and/or a Vaccinium vitis-idaea extract, or a solution containing these extracts in the aforementioned solvent may be mixed with reduced coenzyme Q10, or a solution containing reduced coenzyme Q10 and a solution containing the extracts may be mixed.

Alternatively, reduced coenzyme Q10 obtained by the aforementioned production method of the present invention can be directly utilized, that is, a mixture of reduced coenzyme Q10 after completion of the reduction reaction and, and an acerola extract and/or a Vaccinium vitis-idaea extract can be directly utilized as the composition of the present invention, and this embodiment is one of the most preferable embodiments.

In the composition of the present invention, as a substance other than reduced coenzyme Q10, an acerola extract and/or a Vaccinium vitis-idaea extract, and solvent and surfactant where necessary, for example, excipient, disintegrant, lubricant, binder, dye, anticoagulant, absorption promoter, solubilizing agent, stabilizer, flavor,and active ingredient other than reduced coenzyme Q10 can also be further contained, without any particular limitation. Specific examples, detailed kind and preferable examples thereof are the same as those explained for the stabilization method of the present invention.

The content of an acerola extract and/or a Vaccinium vitis-idaea extract (total amount when the both are used in combination) in the composition of the present invention is 0.1 wt% or more. To allow sufficient exertion of the stabilizing effect of reduced coenzyme Q10, however, the ratio to the total weight of the composition maybe about 0.01 wt% or more (reference), preferably about 1 wt% or more, more preferably about 5 wt% or more. While the upper limit is not particularly set, it is normally about 99 wt% or less, preferably about 95 wt% or less, more preferably about 90 wt% or less, particularly preferably 80 wt% or less, from the economic aspect, effectiveness as a nutrient and the like. According to the invention, the content of reduced coenzyme Q10 in the composition is 0.5 wt% or more. To ensure effectiveness of reduced coenzyme Q10 in the composition, it may be about 0.001 wt% or more, preferably about 0.01 wt% or more, more preferably about 0.1 wt% or more (reference).

While the upper limit is not particularly set, it is preferably about 50 wt% or less, more preferably about 40 wt% or less, particularly preferably about 30 wt% or less.

While the composition of the present invention can be used directly, it can also be used after processing into a preparation as described for the production method of the present invention, such as oral administration forms such as capsules (hard capsule, soft capsule, microcapsule), tablets, syrups, and drinks, and a form for cream, suppository and toothpaste. Of these, processing into the above-mentioned oral administration form is preferable. Particularly preferred is the form of a capsule, particularly a soft capsule. A capsule base material in this case is not particularly limited, and, for example, gelatin derived from beef bones, cattle skin, pig skin, and fish skin, as well as other base materials (e.g., viscosity increasing stabilizers of seaweed-derived products such as carrageenan and alginic acid, vegetable seed-derived products such as locust bean gum and guar gum,
and cellulose-containing agents for production material can also be used.

### Examples

While the present invention is explained in more detail in the following by referring to Examples, the present invention is not limited to those Examples alone. Moreover, the purity of reduced coenzyme Q10, the weight ratio of reduced coenzyme Q10/oxidized coenzyme Q10, and the like in the Examples were measured by the following HPLC analysis. However, the purity of the obtained reduced coenzyme Q10 does not define the critical value of the purity in the present invention, and similarly, the weight ratio of reduced coenzyme Q10 and oxidized coenzyme Q10 does not define the upper limit thereof. To conveniently indicate the weight ratio of reduced coenzyme Q10 and oxidized coenzyme Q10 in the present Example, the ratio of reduced coenzyme Q10 to the total amount of coenzyme Q10 (total amount of oxidized coenzyme Q10 and reduced coenzyme Q10) is shown in percentage as a "weight ratio of reduced coenzyme Q10". For example, "the weight ratio of reduced coenzyme Q10 is 20%" means that the weight ratio of reduced coenzyme Q10 and oxidized coenzyme Q10 is 20/80.

### (HPLC analysis conditions)

column; SYMMETRY C18 (manufactured by Waters) 250 mm (length) 4.6 mm (inner diameter),
mobile phase; C₂H₅OH:CH₃OH=4:3 (v:v),
detection wavelength; 210 nm,
flow rate; 1 ml/min,
retention time of reduced coenzyme Q10; 9.1 min,
retention time of oxidized coenzyme Q10; 13.3 min.

### (Example 1)

Oxidized coenzyme Q10 crystal (0.1 g) and a component derived from a naturally-occurring substance (plant extract, 1.0 g, 10-fold weight) described in Table 1 were respectively added to 99% ethanol (15 g), and the mixture was stirred at 78°C for 16 hr under a nitrogen atmosphere. The weight ratio of reduced coenzyme Q10 in the reaction mixture after the reaction is shown in Table 1.

### (Comparative Example 1)

Oxidized coenzyme Q10 crystal (0.1 g, 0.12 mmol) and the compound described in Table 1 (0.69 mmol) were respectively added to 99% ethanol (15 g), and the mixture was stirred at 78°C for 16 hr under a nitrogen atmosphere. The weight ratio of reduced coenzyme Q10 in the reaction mixture after the reaction is shown in Table 1.

**Table 1**

| reagent name | weight ratio of reduced coenzyme Q10 |
|---|---|
| (Example 1) | |
| acerola extract | 100% |
| Vaccinium vitis-idaea extract | 61% |
| pine bark extract | 48% |
| tea extract | 30% |
| rosemary extract | 14% |
| (Comparative Example 1) | |
| retinol acetate | 4% |
| retinal | 4% |
| carnitine | 0% |
| NADH | 2% |

### (Example 2, Comparative Example 2)

Oxidized coenzyme Q10 crystal (0.1 g) and a component derived from a naturally-occurring substance (plant extract, 1.0 g, 10-fold weight) described in Table 2 or a compound (0.69 mmol) were respectively added to limonene (15 g), and the mixture was stirred at 85°C for 16 hr under a nitrogen atmosphere. The weight ratio of reduced coenzyme Q10 in the reaction mixture after the reaction is shown in Table 2.

**Table 2**

| reagent name | weight ratio of reduced coenzyme Q10 |
|---|---|
| (Example 2) | |
| acerola extract | 98% |
| pine bark extract | 30% |
| tea extract | 19% |
| (Comparative Example 2) | |
| grape seed extract | 0% |
| R-dihydrolipoic acid | 1% |
| L-sodium ascorbate | 1% |

### (Example 3, Comparative Example 3)

Oxidized coenzyme Q10 crystal (0.1 g) and a component derived from a naturally-occurring substance described in Table 3 (plant extract, 1.0 g, 10-fold weight) or a compound (0.69 mmol) were respectively added to a mixture of Span 80 (0.12 g), glycerol (0.09 g), Tween 80 (1.49 g) and MCT (0.43 g), and the mixture was stirred at 80°C for 16 hr under a nitrogen atmosphere. The weight ratio of reduced coenzyme Q10 in the reaction mixture after the reaction is shown in Table 3.

**Table 3**

| reagent name | weight ratio of reduced coenzyme Q10 |
|---|---|
| (Example 3) | |
| acerola extract | 98% |
| pine bark extract | 84% |
| tea extract | 33% |
| rosemary extract | 17% |
| (Comparative Example 3) | |
| ginkgo leaf extract | 5% |
| grapes seed extract | 1% |
| retinal | 3% |
| NADH | 0% |
| α-tocopherol | 0% |
| carnitine | 0% |

### (Example 4, Comparative Example 4)

Oxidized coenzyme Q10 crystal (0.1 g) and a component derived from a naturally-occurring substance described in Table 4 (plant extract, 1.0 g, 10-fold weight) were respectively added to a mixture of condensed ricinoleic acid ester (CR-310, manufactured by Sakamoto Yakuhin Kogyo Co., Ltd., 1.25 g) and MCT (1.25 g), and the mixture was stirred at 80°C for 16 hr under a nitrogen atmosphere. The weight ratio of reduced coenzyme Q10 in the reaction mixture after the reaction is shown in Table 4.

**[Table 4]**

| reagent name | weight ratio of reduced coenzyme Q10 |
|---|---|
| (Example 4) | |
| acerola extract | 100% |
| rosemary extract | 12% |
| (Comparative Example 4) | |
| St. John's wort extract | 5% |
| Grape seed extract | 0% |

### (Example 5)

Reduced coenzyme Q10 crystal (0.02 g) and a component derived from a naturally-occurring substance described in Table 5 (plant extract, 0.2 g, 10-fold weight) were respectively added to 99% ethanol (3.0 g), and the mixture was left standing in the air at 25°C for 24 hr. The weight ratio of reduced coenzyme Q10 in the reaction mixture after the preservation is shown in Table 5. In addition, the result without addition of a component derived from a naturally-occurring substance is also shown as a control.

**[Table 5]**

| reagent name | weight ratio of reduced coenzyme Q10 |
|---|---|
| no addition (control) | 38% |
| acerola extract | 75% |
| Vaccinium vitis-idaea extract | 66% |
| pine bark extract | 93% |

### (Example 6)

Reduced coenzyme Q10 crystal (0.02 g) and a component derived from a naturally-occurring substance described in Table 6 (plant extract, 0.2 g, 10-fold weight) were respectively added to a mixture of Span 80 (0.17 g), glycerol (0.13 g), Tween 80 (2.10 g) and MCT (0.61 g), and the mixture was left standing in the air at 25°C for 24 hr. The weight ratio of reduced coenzyme Q10 in the reaction mixture after the preservation is shown in Table 6. In addition, the result without addition of a component derived from a naturally-occurring substance is also shown as a control.

**[Table 6]**

| reagent name | weight ratio of reduced coenzyme Q10 |
|---|---|
| no addition (control) | 53% |
| acerola extract | 100% |
| pine bark extract | 99% |

### (Example 7)

Reduced coenzyme Q10 crystal (0.02 g) and an acerola extract (0.2 g, 10-fold weight) were added to a mixture of condensed ricinoleic acid ester (CR-310, manufactured by Sakamoto Yakuhin Kogyo Co., Ltd., 1.50 g) and MCT (1.50 g), and the mixture was left standing in the air at 25°C for 24 hr. The weight ratio of reduced coenzyme Q10 in the reaction mixture after the preservation is shown in Table 7. In addition, the result without addition of a component derived from a naturally-occurring substance is also shown as a control.

**[Table 7]**

| reagent name | weight ratio of reduced coenzyme Q10 |
|---|---|
| no addition (control) | 48% |
| acerola extract | 97% |

## Claims

1. A production method of reduced coenzyme Q10, comprising reducing oxidized coenzyme Q10 with a component derived from a naturally-occurring substance, wherein the component derived from a naturally-occurring substance is any one or more components selected from the group consisting of an acerola extract, a tea extract, a rosemary extract, a pine bark extract and a Vaccinium vitis-idaea extract.

2. The production method of claim 1, wherein the component derived from a naturally-occurring substance is an acerola extract and/or a Vaccinium vitis-idaea extract.

3. The production method of claim 1 or 2, comprising performing a reduction reaction in the co-presence of an organic solvent, fats, oils, water or a mixture thereof.

4. The production method of claim 3, wherein the organic solvent is at least one kind selected from the group consisting of alcohols, fatty acids and terpenes.

5. The production method of any one of claims 1 to 4, wherein the concentration of the oxidized coenzyme Q10 in the reaction system at the time of the start of the reduction reaction is 0.01 wt% or more.

6. The production method of any one of claims 1 to 5, wherein the reduction reaction is performed in a preparation.

7. The production method of claim 6, wherein the preparation is a capsule or a soft capsule.

8. The production method of any one of claims 1 to 7, wherein the reduction reaction is performed under a deoxygenation atmosphere.

9. A composition comprising reduced coenzyme Q10, and an acerola extract and/or a Vaccinium vitis-idaea extract, wherein the content of the reduced coenzyme Q10 in the composition is 0.5 wt% or more, and wherein the content of an acerola extract and/or a Vaccinium vitis-idaea extract in the composition is 0.1 wt% or more.

10. The composition of claim 9, further comprising an organic solvent, fats, oils, water or a mixture thereof in the composition.

11. The composition of claim 10, wherein the organic solvent is at least one kind selected from alcohols, fatty acids and terpenes.

12. The composition of any one of claims 9 to 11, wherein the reduced coenzyme Q10 in the composition is externally added.

13. The composition of any one of claims 9 to 11, wherein the reduced coenzyme Q10 in the composition is obtained by reducing oxidized coenzyme Q10 with an acerola extract and/or a Vaccinium vitis-idaea extract.

14. A method of stabilizing reduced coenzyme Q10, comprising placing reduced coenzyme Q10, and an acerola extract and/or a Vaccinium vitis-idaea extract in co-presence.

## Patentansprüche

1. Verfahren zur Herstellung von reduziertem Coenzym Q10, umfassend das Reduzieren von oxidiertem Coenzym Q10 mit einer von einer natürlich auftretenden Substanz abgeleiteten Komponente, wobei die von einer natürlich auftretenden Substanz abgeleiteten Komponente eine oder mehrere Komponenten, ausgewählt aus der Gruppe bestehend aus einem Acerolaextrakt, einem Teeextrakt, einem Rosmarinextrakt, einem Kiefernrindenextrakt und einem Vaccinium-vitis-idaea-Extrakt, sind.

2. Das Verfahren zur Herstellung nach Anspruch 1, wobei die von einer natürlich auftretenden Substanz abgeleiteten Komponente ein Acerolaextrakt und/oder ein Vaccinium-vitis-idaea-Extrakt ist.

3. Das Verfahren zur Herstellung nach Anspruch 1 oder 2, umfassend das Durchführen einer Reduktionsreaktion in gleichzeitiger Anwesenheit eines organischen Lösungsmittels, Fetten, Ölen, Wasser oder eines Gemisches davon.

4. Das Verfahren zur Herstellung nach Anspruch 3, wobei das organische Lösungsmittel mindestens eine Art, ausgewählt aus der Gruppe bestehend aus Alkoholen, Fettsäuren und Terpenen, ist.

5. Das Verfahren zur Herstellung nach einem der Ansprüche 1 bis 4, wobei die Konzentration des oxidierten Coenzyms Q10 im Reaktionssystem bei Beginn der Reduktionsreaktion bei 0,01 Gew.-% oder mehr liegt.

6. Das Verfahren zur Herstellung nach einem der Ansprüche 1 bis 5, wobei die Reduktionsreaktion in einer Zubereitung durchgeführt wird.

7. Das Verfahren zur Herstellung nach Anspruch 6, wobei die Zubereitung eine Kapsel oder eine Weichkapsel ist.

8. Das Verfahren zur Herstellung nach einem der Ansprüche 1 bis 7, wobei die Reduktionsreaktion unter einer Atmosphäre, aus der Sauerstoff entfernt wurde, durchgeführt wird.

9. Eine Zusammensetzung, umfassend reduziertes Coenzym Q10, und ein Acerola-Extrakt und/oder ein Vaccinium-vitis-idaea-Extrakt, wobei der Gehalt an reduziertem Coenzym Q10 in der Zusammensetzung 0,5 Gew.-% oder mehr beträgt, und wobei der Gehalt an Acerolaextrakt und/oder an Vaccinium-vitis-idaea-Extrakt in der Zusammensetzung 0,1 Gew-% oder mehr beträgt.

10. Die Zusammensetzung nach Anspruch 9, weiter umfassend ein organisches Lösungsmittel, Fette, Öle, Wasser oder ein Gemisch davon in der Zusammensetzung.

11. Die Zusammensetzung nach Anspruch 10, wobei das organische Lösungsmittel mindestens eine Art, ausgewählt aus Alkoholen, Fettsäuren und Terpenen, ist.

12. Die Zusammensetzung nach einem der Ansprüche 9 bis 11, wobei das reduzierte Coenzym Q10 in der Zusammensetzung von außen zugegeben wird.

13. Die Zusammensetzung nach einem der Ansprüche 9 bis 11, wobei das reduzierte Coenzym Q10 in der Zusammensetzung erhalten wird durch Reduzieren von oxidiertem Coenzym Q10 mit einem Acerolaextrakt und/oder Vaccinium-vitis-idaea-Extrakt.

14. Ein Verfahren zur Stabilisierung von reduziertem Coenzym Q10, umfassend das Bereitstellen der gleichzeitigen Anwesenheit von reduziertem Coenzym Q10 und einem Acerolaextrakt und/oder Vaccinium-vitis-idaea-Extrakt.

## Revendications

1. Procédé de production de coenzyme Q10 réduite, comprenant la réduction de la coenzyme Q10 oxydée avec un composant dérivé d'une substance existant à l'état naturel, dans lequel le composant dérivé d'une substance existant à l'état naturel est l'un quelconque ou plusieurs composants choisis dans le groupe constitué d'un extrait d'acérola, un extrait de thé, un extrait de romarin, un extrait d'écorce de pin et un extrait de Vaccinium vitis-idaea.

2. Procédé de production selon la revendication 1, dans lequel le composant dérivé d'une substance existant à l'état naturel est un extrait d'acérola et/ou un extrait de Vaccinium vitis-idaea.

3. Procédé de production selon la revendication 1 ou 2, comprenant la réalisation d'une réaction de réduction en coprésence d'un solvant organique, de graisses, d'huiles, d'eau ou de l'un de leurs mélanges.

4. Procédé de production selon la revendication 3, dans lequel le solvant organique est au moins un type choisi dans le groupe constitué d'alcools, d'acides gras et de terpènes.

5. Procédé de production selon l'une quelconque des revendications 1 à 4, dans lequel la concentration de la coenzyme Q10 oxydée dans le système réactionnel au moment du départ de la réaction de réduction est de 0,01 % en poids ou supérieure.

6. Procédé de production selon l'une quelconque des revendications 1 à 5, dans lequel la réaction de réduction est réalisée dans une préparation.

7. Procédé de production selon la revendication 6, dans lequel la préparation est une capsule ou une capsule molle.

8. Procédé de production selon l'une quelconque des revendications 1 à 7, dans lequel la réaction de réduction est réalisée sous une atmosphère de désoxygénation.

9. Composition comprenant de la coenzyme Q10 réduite, et un extrait d'acérola et/ou un extrait de Vaccinium vitis-idaea, dans laquelle la teneur en coenzyme Q10 réduite dans la composition est de 0,5 % en poids ou supérieure, et dans laquelle la teneur en un extrait d'acérola et/ou un extrait de Vaccinium vitis-idaea dans la composition est de 0,1 % en poids ou supérieure.

10. Composition selon la revendication 9, comprenant en outre un solvant organique, des graisses, des huiles, de l'eau ou l'un de leurs mélanges dans la composition.

11. Composition selon la revendication 10, dans laquelle le solvant organique est au moins un type choisi parmi des alcools, des acides gras et des terpènes.

12. Composition selon l'une quelconque des revendications 9 à 11, dans laquelle la coenzyme Q10 réduite dans la composition est ajoutée en externe.

13. Composition selon l'une quelconque des revendications 9 à 11, dans laquelle la coenzyme Q10 réduite dans la composition est obtenue en réduisant de la coenzyme Q10 oxydée avec un extrait d'acérola et/ou un extrait de Vaccinium vitis-idaea.

14. Procédé de stabilisation de la coenzyme Q10 réduite, comprenant le placement de la coenzyme Q10 réduite, et d'un extrait d'acérola et/ou d'un extrait de Vaccinium vitis-idaea en coprésence.
